# EUROPEAN PATENT APPLICATION

(11) **EP 3 305 274 A1**
(43) Date of publication of application: **11.04.2018**
(21) Application number: 15893599.9
(22) Date of filing: 29.05.2015
(51) Int. Cl.: A61K 8/44, C12P 13/04

(54) **PREPARATION CONTAINING ERGOTHIONEINE, PREPARATION METHOD THEREOF AND USE OF MUSHROOM EXTRACELLULAR FERMENT LIQUOR**

(71) Applicant: Tianjin Institute Of Industrial Biotechnology, Chinese Academy of Sciences, Tianjin 300308 (CN); Tianjin Sinonocy Biological Technology Co., Ltd., Binhai Hi-tech Industrial Development Area, Tianjin 300301 (CN)
(72) Inventor: JIANG, Wenxia, Tianjin 300308 (CN); YANG, Ping, Tianjin 300308 (CN); LIU, Qi, Tianjin 300308 (CN); ZHANG, Weiya, Tianjin 300308 (CN); ZHOU, Tao, Tianjin 300308 (CN); MEI, Baoliang, Tianjin 300308 (CN); ZHOU, Zizhen, Tianjin 300301 (CN); LI, Miao, Tianjin 300301 (CN); ZHANG, Dalong, Tianjin 300301 (CN)
(74) Representative: Ström & Gulliksson AB
(86) International application number: PCT/CN2015/080269
(87) International publication number: WO 2016/191936

(57) **Abstract**

Disclosed is a preparation containing ergothioneine, the ergothioneine is provided by an extracellular ferment liquor which is obtained by removing the mycelia from the ferment liquor produced through liquid fementation of a mushroom and/or the concentrate of the extracellular ferment liquor, and the preparation comprises food, cosmetics and animal feed. The method for preparing the preparation comprises liquid fermentation of a mushroom, removing the mycelia from the fermentation product, and selectively concentrating the extracellular ferment liquor obtained after removing the mycelia. The extracellular ferment liquor obtained through the liquid fermentation of a mushroom and/or the concentrate of the extracellular ferment liquor are used in preparing the preparation containing ergothioneine.

## Description

### Field of the Invention

The present invention relates to a preparation containing ergothioneine, a method for preparing the same, and a use of mushroom extracellular ferment liquor, in particular to a preparation containing ergothioneine, a method for preparing the same, and a use of mushroom extracellular ferment liquor for preparing the preparation containing ergothioneine.

### Background of the Invention

Ergothioneine (L-Ergothioneine, EGT) is the only known natural 2-thio-imidazole-amino acid up to now and is a rare natural chiral amino acid. It has efficacies including maintaining oxidation-reduction equilibrium in the system, detoxifying exogenous substances, preventing oxidative damages and radiation damages, and preventing cancer, etc., in human body. Though the blood of human contains ergothioneine at concentration of about 1-4mg per 100mL blood, ergothioneine can't be synthesized in human body itself, and has to be ingested from food instead. Ergothioneine can be synthesized in fungi, which are an important source of ergothioneine for animals and plants.

Though ergothioneine can be prepared via chemical synthesis method, both the difficulty and the cost of chemical synthesis method are very high owing to the fact that ergothioneine is a chiral compound. If ergothioneine is prepared via submerged fermentation of mycelia of edible fungi, the product will be highly safe, and the content of ergothioneine in the fermentation product can be increased significantly with a metabolism regulation and submerged fermentation control strategy. Such a fermentation measure is obviously better than techniques of extracting ergothioneine from pig blood, animal tissues, fruit bodies of edible fungi, ergots and grains.

It is reported in literatures that ergothioneine synthesized by mushroom is accumulated in mycelia (Wi Young Lee, Eung-Jun Park, Jin Kwon Ahn. Supplementation of Methionine Enhanced the Ergothioneine Accumulation in the Ganoderma Neo-Japonicum Mycelia [J]. Appl Biochem Biotechnol, 2009, 158: 213-221.; Pramvadee Tepwong, Anupam Giri, Fumito Sasaki, et al. Mycobial Enhancement of Ergothioneine by Submerged Cultivation of Edible Mushroom Mycelia and Its Application as an Antioxidative Compound [J]. Food Chemistry, 2012, 131: 247-258); to measure the content of ergothioneine in the mycelia or extract ergothioneine from the mycelia, an extraction or leaching method should be used to extract or leach the ergothioneine in the mycelia out of the cells. In relevant researches, ergothioneine is usually extracted with an organic solvent; for example, Huynh N. D. Bao and Pramvadee Tepwong used 70% ethanol to leach ergothioneine in the mycelia of *Flammulina velutipes* or *Lentinus edodes* (Pramvadee Tepwong, Anupam Giri, Fumito Sasaki, et al. Mycobial Enhancement of Ergothioneine by Submerged Cultivation of Edible Mushroom Mycelia and Its Application as an Antioxidative Compound [J]. Food Chemistry, 2012, 131: 247-258.; Huynh N.D. Bao, Yoichi Shinomiya, Hiroaki Ikeda, et al. Preventing Discoloration and Lipid Oxidation in Dark Muscle of Yellowtail by Feeding an Extract Prepared from Mushroom (Flammulina Uelutipes) Cultured Medium [J]. Aquaculture, 2009, 295: 243-249); Wi Young Lee utilized 70% ethanol solution that contains a surfactant to leach ergothioneine in *Ganoderma Neo-japonicum* Mycelia (Wi Young Lee, Eung-Jun Park, Jin Kwon Ahn. Supplementation of Methionine Enhanced the Ergothioneine Accumulation in the Ganoderma Neo-japonicum Mycelia [J]. Appl Biochem Biotechnol, 2009, 158: 213-221); Nianbo ZHOU utilized NaOH solution with pH=8.0 and boiling water to leach ergothioneine in mushroom handle of *Agaricus bisporus* (Nianbo ZHOU, Yiqun LI, Qinhong YIN, Separation of Ergothioneine from Mushroom Handle of Agaricus Bisporus by Alumina Column Chromatography [J]. Journal of Anhui Agricultural Science, 2010, 38(27): 14842-14843.). CN103181933A and CN103743825A have also disclosed leaching ergothioneine from mycelia with an aqueous leaching method, which avoids the possible risks incurred by an organic solvent to the production process and product. However, there is no report on how to utilize the liquid portion of the fermentation broth obtained through solid-liquid separation.

### Summary of the Invention

To overcome the drawbacks in the prior art, the present invention provides a preparation containing ergothioneine, a method for preparing the same, and a use of mushroom extracellular ferment liquor.

Based on the report in existing literatures, for those skilled in the art, it is a general cognition that the intracellular ergothioneine would not be secreted out of the cells, or the content of ergothioneine in the secreta would be very low (too low to be detected or utilized) even if the ergothioneine can be secreted out of the cells. However, the inventor of the present invention has found during the research: the extracellular ferment liquor of mushroom obtained through shaking-flask fermentation contains ergothioneine at a certain concentration (7mg/L or more), and the content of ergothioneine in the extracellular ferment liquor can be further increased by improving the fermentation condition (e.g., the content of ergothioneine in the extracellular ferment liquor obtained through fermentation in a 75L fermenter can be as high as 61.7mg/L). Therefore, to attain the object described above, in a first aspect, the present invention provides a preparation containing ergothioneine, wherein the ergothioneine is provided by an extracellular ferment liquor that is obtained by removing the mycelia from a ferment liquor produced through liquid fermentation of mushroom, and/or a concentrate of the extracellular ferment liquor, the preparation comprises food, cosmetics and animal feed.

In a second aspect, the present invention provides a method for preparing the preparation described in the first aspect, which comprises liquid fermentation of mushroom, removing the mycelia from the obtained ferment liquor, and selectively concentrating the extracellular ferment liquor obtained after removing the mycelia.

In a third aspect, the present invention provides a use of an extracellular ferment liquor obtained through liquid fermentation of mushroom and/or a concentrate of the extracellular ferment liquor in preparing a preparation containing ergothioneine, wherein the preparation comprises food, cosmetics and animal feed.

The present invention utilizes extracellular ferment liquor that contains rich ergothioneine to prepare a preparation containing ergothioneine. The method provided in the present invention not only makes the most of the ergothioneine outside the mycelia, but also omits leaching step, and the process is simpler.

Other features and advantages of the present invention will be further detailed in the embodiments hereunder.

### Detailed Description of the Embodiments

Hereunder some embodiments of the present invention will be detailed. It should be understood that the embodiments described here are only provided to describe and explain the present invention, but shall not be deemed as constituting any limitation to the present invention.

In the present invention, unless otherwise specified, the term "ferment liquor" refers to a liquid fermentation product of mushroom (containing mycelia); "extracellular ferment liquor" refers to the portion of the ferment liquor that doesn't contain mycelia, i.e., a product obtained by removing the mycelia from the ferment liquor obtained through liquid fermentation of mushroom; "saturation of dissolved oxygen" is an indicator that indicates the content of dissolved oxygen, and the higher the value of the indicator is, the higher the content of dissolved oxygen is; saturation of dissolved oxygen (%) = (measured content of dissolved oxygen / saturated content of dissolved oxygen under the measuring condition) × 100%.

According to a first aspect of the present invention, in the preparation containing ergothioneine provided in the present invention, the ergothioneine is provided by an extracellular ferment liquor that is obtained by removing the mycelia from a ferment liquor produced through liquid fermentation of a mushroom, and/or a concentrate of the extracellular ferment liquor, wherein the preparation comprises food, cosmetics and animal feed. Wherein the content of ergothioneine in extracellular ferment liquor is preferably 7-62mg/L.

In the present invention, the mushroom may be ordinary mushroom from *Tricholomataceae,* such as mushroom from *Pleurotus.* Preferably, the mushroom is *Pleurotus ostreatus,* such as *Pleurotus ostreatus* with preservation number CGMCC No. 6232 (preserved in China General Microbiological Culture Collection Center (CGMCC) on June 15, 2012, and disclosed in CN103734022A).

In the present invention, there is no particular restriction on the liquid culture medium used for the liquid fermentation. The liquid culture medium may contain 10-95g/L glycerol, 10-80g/L casein peptone, 1-6g/L KH₂PO₄, and 0.2-5g/L MgSO₄. However, the inventor of the present invention has found that the content of ergothioneine in the extracellular ferment liquor can be further increased by adding specific amino acids to the liquid culture medium. Therefore, according to a preferred embodiment of the present invention, the liquid culture medium used for the liquid fermentation contains 10-95g/L glycerol, 10-80g/L casein peptone, 1-6g/L KH₂PO₄, 0.2-5g/L MgSO₄, 3-45mmol/L methionine, and 2-45mmol/L cysteine. More preferably, the liquid culture medium used for the liquid fermentation contains 65-95g/L glycerol, 40-80g/L casein peptone, 2-4g/L KH₂PO₄ and 0.5-2g/L MgSO₄, 9-35mmol/L methionine and 5-20mmol/L cysteine. According to an optimal embodiment of the present invention, the liquid culture medium used for the liquid fermentation contains 75g/L glycerol, 50g/L casein peptone, 3g/L KH₂PO₄ and 1.5g/L MgSO₄, 14mmol/L methionine and 7.5mmol/L cysteine.

According to a preferred embodiment of the present invention, the condition of liquid fermentation includes: fermentation temperature: 19-31°C, fermentation time: 7-15 days, and saturation of dissolved oxygen: 10-50%. According to a further preferred embodiment of the present invention, the condition of liquid fermentation includes: fermentation temperature: 23-28°C, fermentation time: 10-13 days, and saturation of dissolved oxygen: 15-40%.

By utilizing *Pleurotus ostreatus* as the fermentation fungus and selecting the above-mentioned liquid culture medium and liquid fermentation condition, the content of ergothioneine in the extracellular ferment liquor can be further increased.

In the preparation provided in the present invention, the content of ergothioneine may be in a wide range; for example, the content of ergothioneine may be 0.0005-50wt%, preferably 0.001-20wt%, based on the total weight of the preparation.

The method for removing the mycelia from the ferment liquor may be a conventional method, such as solid-liquid separation (e.g., filtering, centrifugation or precipitation). As described above, the extracellular ferment liquor may be a liquid phase obtained from the liquid fermentation product through solid-liquid separation.

In the present invention, to improve the content of ergothioneine in the preparation, a concentrate of extracellular ferment liquor may be used to provide ergothioneine. The concentrate that provides ergothioneine for the preparation may be in a viscous state, or may be a solution, a suspension, an emulsion, or a paste, or a powder or a solid in any other form.

The activation, seed culturing, liquid fermentation and liquid culture medium, etc., involved for obtaining the extracellular ferment liquor are not limited, and may be selected alternatively as described in the following text.

The preparation provided in the present invention may be food, cosmetics and animal feed, in which ergothioneine should be added as an additive or active ingredient (or effective ingredient) to meet different demands. There is no particular restriction on the specific form of the preparation. The preparation may be liquid, solid or paste.

In the present invention, the food may comprise meal (food that must be ingested in daily life, i.e., staple food), functional food, and leisure food, etc.

The meal may comprise various grain staple foods, such as foods prepared from wheat, rice, potato, or sweet potato, etc., e.g., steamed bread, steamed stuffed bun, dumpling, noodle, vermicelli, bread, and cake, etc.

The functional food may be functional beverage, capsule, or tablet. In fact, there is no particular requirement for the specific form of the function food. The functional food may be in any form of common oral preparation, such as decoction, pill, powder, paste, pellet, liquor, granule, effervescent, oral liquid, capsule, tablet or injection, etc. In consideration of process simplification, preferably the functional food is a form of functional beverage.

The leisure food may comprise dry fruit, puffed food, candy, meat food, and beverage, etc., such as potato chips, French fries, prawn chips, crackers, preserved fruit, preserved plums, peanuts, pine nuts, almonds, pistachio nuts, fish fillets, jerky, spiced fried meat, and fruit juice, etc.

In the present invention, the cosmetics may be cosmetics in any form, such as lotion, makeup remover, perfume, cologne, floral water, emulsion, cream, facial mask, base cream, cream foundation, lipstick, toothpaste, dentifrice, bath foam, shampoo, hair conditioner, perfume soap, or laundry soap, etc.

In the present invention, the animal feed may be any feed or nutritional supplement that provides nutrients to any animal in any growth stage.

According to a specific form of preparation described above, those skilled in the art can select other constituents in the preparation. For example, in the case that the preparation is a functional beverage, the preparation may further contain at least one of sweetener, thickener, preservative, and acidity regulator (e.g., carboxymethyl cellulose, phospholipid, aspartame, xylitol, essence, xanthan gum, sucrose and citric acid, etc.). Such a selection will not be further detailed here.

The amount of usage (dose) of the preparation provided in the present invention may be selected according to the purpose of the preparation and the demand of the user. For example, in the case that the preparation is a functional food, usually, measured in the weight of ergothioneine, the dose of the preparation provided in the present invention may be 20µg/kg body weight/day to 3mg/kg body weight/day.

According to a second aspect of the present invention, the method for preparing the preparation described in the first aspect comprises liquid fermentation of the mushroom, removing the mycelia from the obtained ferment liquor, and selectively concentrating the extracellular ferment liquor obtained after removing the mycelia (to obtain a concentrate of the extracellular ferment liquor). A variety of preparations that contain ergothioneine may be obtained by mixing the extracellular ferment liquor obtained by liquid fermentation of the mushroom and removing the mycelia from the ferment liquor and/or concentrate of the extracellular ferment liquor with other required constituents. Wherein the specific selection or condition of the mushroom and liquid fermentation have been described above, and will not be further describe here.

In the present invention, the method may further comprise activating the mushroom and carrying out seed culturing before the liquid fermentation, wherein the activation is to culture the fungus in preserved state in an appropriate culture medium to revitalize the fungus; the seed culturing is to obtain more pure and vigorous culture, i.e., obtain mycelia that are vigorous and in a quantity enough for inoculation. The activation and seed culturing may be carried out with a conventional method in the art. For example, the activation may comprise inoculating the mycelia of the mushroom onto a PDA slant and culturing for 5-9 days at 23-28°C. The seed culturing may comprise inoculating the activated fungus into a seed culture medium (liquid) and culturing for 3-6 days at 23-28°C. The PDA slant and the seed culture medium are well known to those skilled in the art, and will not be further detailed here.

In a preferred embodiment of the present invention, the method for liquid fermentation of the mushroom comprises the following steps:
(a) inoculating *Pleurotus ostreatus* into a seed culture medium, and culturing for 3-6 days at 23-28°C to prepare a seed liquor, wherein the seed culture medium contains 25-40g/L maize meal, 15-35g/L bean cake powder, 30-80U/L alpha-amylase, 2-4.5g/L potassium dihydrogen phosphate, and 0.2-3g/L magnesium sulfate; and
(b) inoculating the seed liquor in an inoculum size of 4-20vol% into a liquid culture medium, and culturing for 7-15 days (preferably 10-13 days) at 19-31°C (preferably 23-28°C), and controlling the saturation of dissolved oxygen at 10-50% (preferably 15-40%) during the culturing process, to obtain a ferment liquor that contains *Pleurotus ostreatus* mycelia, wherein the liquid culture medium contains 65-95g/L glycerol, 40-80g/L casein peptone, 2-4g/L potassium dihydrogen phosphate, 0.5-2g/L magnesium sulfate, 9-35mmol/L methionine, and 5-20mmol/L cysteine.

In a further preferred embodiment of the present invention, the method for liquid fermentation of the mushroom comprises the following steps:
(a) inoculating *Pleurotus ostreatus* into a seed culture medium, and culturing for 4 days at 25°C to prepare a seed liquor, wherein the seed culture medium contains 30g/L maize meal, 15g/L bean cake powder, 80U/L alpha-amylase, 3g/L potassium dihydrogen phosphate, and 1.5g/L magnesium sulfate; and
(b) inoculating the seed liquor in an inoculum size of 5vol% into a liquid culture medium, culturing for 3 days at 25°C and then adjusting the pH to 6, and culturing for 12 days at 31°C, and controlling the saturation of dissolved oxygen at 30% during the culturing process, to obtain a ferment liquor that contains *Pleurotus ostreatus* mycelia, wherein the liquid culture medium contains 75g/L glycerol, 50g/L casein peptone, 3g/L potassium dihydrogen phosphate, 1.5g/L magnesium sulfate, 14mmol/L methionine, and 7.5mmol/L cysteine.

According to an optimal embodiment of the present invention, the liquid culture medium further contains tween (e.g., tween 60 and/or tween 80). The dose of the tween may be 0.5-50g/L; for example, tween 60: 2-50g/L (preferably 5-40g/L, more preferably 5g/L), tween 80: 0.5-40g/L (preferably 5-40g/L, more preferably 10g/L). The content of ergothioneine in the extracellular ferment liquor can be further increased remarkably by adding tween.

To obtain a preparation that has higher content of ergothioneine, the extracellular ferment liquor may be concentrated alternatively in an appropriate way, and the condition of concentration may include: vacuum degree: 0-0.1MPa.

In the present invention, the method may further comprise sterilization of the extracellular ferment liquor obtained by removing mycelia (or a concentrate of the extracellular ferment liquor). Then, other constituents required for the preparation can be mixed with the sterilized extracellular ferment liquor (or the concentrate of the extracellular ferment liquor).

According to the third aspect of the present invention, the present invention provides a use of an extracellular ferment liquor obtained through liquid fermentation of a mushroom (preferably *Pleurotus ostreatus)* and/or a concentrate of the extracellular ferment liquor in preparing a preparation containing ergothioneine, wherein the preparation comprises food, cosmetics and animal feed. The extracellular ferment liquor and/or the concentrate of the extracellular ferment liquor obtained through liquid fermentation of the mushroom may be the extracellular ferment liquor (or the concentrate of the extracellular ferment liquor) described above, and the specific form of the preparation may be a form described above too, and will not be detailed further here.

Hereunder the present invention will be detailed in examples. In the following examples, the experimental instruments are as follows:
Fungus: *Pleurotus ostreatus,* Preservation No. is CGMCC No. 6232.

Experimental instruments and/or materials: shaking table (IS-RDV3, Crystal Technology & Industries, Inc., USA); 75L fermenter (BIOSTAT D-DCU75, Sartorius); high-performance liquid chromatograph (Agilent 1260, Agilent Technologies); water bath (TW20, Julabo); magnetic stirrer (WHMIXdrive6, WIGGENS) and controller (MIXcontrol 20, WIGGENS); electronic analytical balance (AB204-S, METTLER TOLEDO); high-speed desktop-type refrigerated centrifuge (TGL-16M, Xiangyi Centrifuge Instrument Co., Ltd.); vacuum drying oven (VOS-60A, STIK); piston-type vacuum pump (V610, ChemVak); ultrasonic wave cleaner (SB-5200D, Ningbo Scientz Biotechnology Inc.); 0.22µm millipore filter (Tianjin Bonna-Agela Technologies, Co., Ltd.); ultrafiltration centrifuge tube (0.5mL, 3kDa, Millipore).

Main reagents: ergothioneine reference sample (purity≤98%, Biomol International Inc.); PDA culture medium (Becton, Dickinson and Company); methanol and other reagents are chromatographically pure commercial products; potassium dihydrogen phosphate and magnesium sulfate are purchased from Sinophram Chemical Reagent Co., Ltd.; maize meal is purchased from Meihekou Xingda Cereals Industry Co., Ltd.; bean pulp powder and bean cake powder are purchased from Zhongmian Ziguang Biotechnology Co., Ltd.; glycerol is purchased from Tianjin Fengchuang Chemical Reagent Technologies Co., Ltd.; casein peptone is purchased from Yanshi Baijia Industry and Trade Co., Ltd.

### Example 1: Shaking-flask fermentation experiment of ergothioneine

Seed culture medium: 30g/L maize meal, 15g/L bean cake powder, 80U/L alpha-amylase, 3g/L potassium dihydrogen phosphate, 1.5g/L magnesium sulfate, and the rest is water; the seed culture medium is sterilized for 20min at 121°C, and 150mL seed culture medium is loaded into a 500mL triangular flask.

Liquid culture medium: 50g/L glycerol, 35g/L casein peptone, 3g/L potassium dihydrogen phosphate, 1.5g/L magnesium sulfate, and the rest is water; the liquid culture medium is sterilized at 20min for 121°C, and 150mL liquid culture medium is loaded into a 500mL triangular flask.

The lawn of PDA slant culture is selected and inoculated into the seed culture medium, and shake cultured for 4 days at 25°C, 150rpm, to obtain a seed liquor. The seed liquor is inoculated in an inoculum size of 5vol% into the liquid culture medium, and shake cultured for 15 days at 25°C, 150rpm, to obtain ferment liquor that contains mycelia.

The ferment liquor of ergothioneine is filtered through a piece of cloth. The filtrate is the extracellular ferment liquor, and the content of ergothioneine in the extracellular ferment liquor is 7.14mg/L.

Besides, 14mmol/L methionine and 7.5mmol/L cysteine are added to the liquid culture medium additionally and shaking-flask fermentation is carried out. The content of ergothioneine in the obtained extracellular ferment liquor is 9.28mg/L. Thus, apparently the content of ergothioneine in the extracellular ferment liquor can be further increased by adding amino acids.

The content of ergothioneine in the extracellular ferment liquor is measured with the following method:

### (1) Pretreatment before measurement of the content of ergothioneine in the extracellular ferment liquor

The extracellular ferment liquor is loaded into a ultrafiltration centrifuge tube with 3kDa of molecular weight cutoff, and centrifuged for 10min under 12840×g, and the permeate liquid is collected; thus, a sample for measuring the content of ergothioneine in the extracellular ferment liquor is obtained.

### (2) HPLC measurement

Preparation of a reference solution: 10mg ergothioneine reference sample is weighed accurately, and mixed with pure water in a 25mL volumetric flask to prepare a reference stock solution at a concentration of 400mg/L. An appropriate amount of the stock solution is taken accurately and mixed with pure water to prepare solutions at a concentration of 40mg/L, 80mg/L, 120mg/L, 160mg/L and 200mg/L respectively, and the solutions are filtered through a 0.22µm millipore filter, so as to obtain reference solutions.

Qualitative and quantitative measurement: HPLC measurement is carried out for the reference solutions of ergothioneine and the samples under the same chromatographic condition, the chromatogram of the sample is compared with the chromatogram of the reference solution of ergothioneine, and the chromatogram peak of ergothioneine in the sample is determined according to the retention time. A standard curve of concentration of the reference solution of ergothioneine vs. the corresponding peak area is plotted, quantitative measurement is carried out with an external standard method under a condition that the sample size of the reference solution is the same as the sample size of the tested sample, and the content of ergothioneine in the tested sample is calculated. The concentrations of the sample at SNR (ratio of peak height of sample to peak height of noise) = 3 and 10 are defined as detection limit and quantitative measurement limit for the detection method, i.e., 10.4µg/L and 34.7µg/L respectively.

Condition of detection: the chromatographic columns are Agilent Eclipse XDB-C18 (4.6×250mm, 5µm), and two chromatographic columns are used in series; the flowing phase is 1% methanol solution, and the pH of the flowing phase is adjusted to 5.0 with acetic acid-sodium acetate buffer solution; the detection wavelength is 257nm; the flow velocity is 0.7mL/min.; the column temperature is 25°C; the sample size is 5µL.

### Example 2: Influence of tween 60 on the content of ergothioneine in the extracellular ferment liquor

Shaking-flask fermentation is carried out with the method described in example 1, but 5g/L, 10g/L, 20g/L and 40g/L tween 60 are added respectively to the liquid culture medium additionally, and the liquid culture medium in which no tween 60 is added is used as a reference group. The mycelia of *Pleurotus ostreatus* are fermented and cultured, and the content of ergothioneine in the extracellular ferment liquor is measured after the fermentation. It is found that the content of ergothioneine in the extracellular ferment liquor is increased by adding tween 60 in a concentration of 5g/L, 10g/L, 20g/L, or 40g/L; wherein the content of ergothioneine in the extracellular ferment liquor is the highest (as high as 16.24mg/L) when 5g/L tween 60 is added, and is increased by 86% compared with the content of ergothioneine in the reference group (8.73mg/L).

### Example 3: Influence of tween 80 on the content of ergothioneine in the extracellular ferment liquor

Shaking-flask fermentation is carried out with the method described in example 1, but 5g/L, 10g/L, 20g/L and 40g/L tween 80 are added respectively to the liquid culture medium additionally, and the liquid culture medium in which no tween 80 is added is used as a reference group. The mycelia of *Pleurotus ostreatus* are fermented and cultured, and the content of ergothioneine in the extracellular ferment liquor is measured after the fermentation. It is found that the content of ergothioneine in the extracellular ferment liquor is increased by adding tween 80 in a concentration of 5g/L, 10g/L, 20g/L, or 40g/L; wherein the content of ergothioneine in the extracellular ferment liquor is the highest (as high as 18.72mg/L) when 10g/L tween 80 is added, and is increased by 114.4% compared with the content of ergothioneine in the reference group (8.73mg/L).

### Example 4: Fermentation experiment of ergothioneine in a 75L fermenter

Seed culture medium for shaking-flask fermentation: 30g/L maize meal, 15g/L bean cake powder, 80U/L alpha-amylase, 3g/L potassium dihydrogen phosphate, 1.5g/L magnesium sulfate, and the rest is water; 300mL seed culture medium is loaded into a 1L triangular flask, and the seed culture medium is sterilized for 20min at 121°C.

Liquid culture medium in 75L fermenter: 75g/L glycerol, 50g/L casein peptone, 3g/L potassium dihydrogen phosphate, 1.5g/L magnesium sulfate, 14mmol/L methionine, 7.5mmol/L cysteine, and the rest is water; 45.6L liquid culture medium is loaded in the fermenter, and the liquid culture medium is sterilized at 20min for 121°C.

The lawn of PDA slant culture is selected and inoculated into the seed culture medium, and shake cultured for 4.5 days at 25°C, 150rpm, to obtain a seed liquor. The seed liquor is inoculated in an inoculum size of 6.6vol% into the fermenter, and cultured for 12.5 days at 25°C, 0.1MPa pressure in the fermenter, the saturation of dissolved oxygen is controlled at 30% during the fermentation process. The measured content of ergothioneine in the extracellular ferment liquor is 61.7mg/L.

### Example 5

Liquid fermentation is carried out with the method described in example 4, but the liquid culture medium consists of 10g/L glycerol, 45g/L casein peptone, 2g/L potassium dihydrogen phosphate, 0.2g/L magnesium sulfate, 25mmol/L methionine, 10mmol/L cysteine, and the rest is water; 48L liquid culture medium is loaded into a 75L fermenter, and the liquid culture medium is sterilized for 20min at 121°C.

The content of ergothioneine in the obtained extracellular ferment liquor is 20.4mg/L.

### Example 6

Liquid fermentation is carried out with the method described in example 4, but the liquid culture medium consists of 95g/L glycerol, 80g/L casein peptone, 4.5g/L potassium dihydrogen phosphate, 5g/L magnesium sulfate, 3mmol/L methionine, 45mmol/L cysteine, and the rest is water; 48L liquid culture medium is loaded into a 75L fermenter, and the liquid culture medium is sterilized for 20min at 121°C.

The content of ergothioneine in the obtained extracellular ferment liquor is 22.1mg/L.

### Example 7

Liquid fermentation is carried out with the method described in example 4, but the liquid culture medium consists of 50g/L glycerol, 35g/L casein peptone, 2g/L potassium dihydrogen phosphate, 2.5g/L magnesium sulfate, 45mmol/L methionine, 2mmol/L cysteine, and the rest is water; 48L liquid culture medium is loaded into a 75L fermenter, and the liquid culture medium is sterilized for 20min at 121°C.

The content of ergothioneine in the obtained extracellular ferment liquor is 27.2mg/L.

### Example 8

This example is provided to describe the method for preparing a preparation (a functional beverage) in the present invention.
(1) The following raw materials are weighed: 800mL extracellular ferment liquor prepared in example 1, 0.2g carboxymethyl cellulose (stabilizer), 0.2g phospholipid, 0.5g aspartame (corrective), 30g xylitol, 3g essence, and 200mL purified water.
(2) The carboxymethyl cellulose, phospholipid, aspartame, and xylitol are mixed homogeneously, the mixture is dissolved in a small amount of water, and then the extracellular ferment liquor prepared in example 1 and the remaining water are added therein; then the mixture is milled twice in a colloid mill to homogeneous state, and then the essence is added.
(3) The material obtained in step (2) is packed, and then sterilized for 20min at 121°C.

### Example 9

This example is provided to describe the method for preparing a preparation (a functional beverage) in the present invention.
(1) The following raw materials are weighed: 800mL extracellular ferment liquor prepared in example 2 (5g/L tween 60 is added), 0.2g carboxymethyl cellulose (stabilizer), 0.2g phospholipid, 0.5g aspartame (corrective), 30g xylitol, 3g essence, and 200mL purified water.
(3) The carboxymethyl cellulose, phospholipid, aspartame, and xylitol are mixed homogeneously, the mixture is dissolved in a small amount of water, and then the extracellular ferment liquor prepared in example 2 and the remaining water are added therein; then the mixture is milled twice in a colloid mill to homogeneous state, and then the essence is added.
(4) The material obtained in step (3) is pasteurized for 30min at 85°C, and then packed in hot state.

### Example 10

This example is provided to describe the method for preparing a preparation (a functional beverage) in the present invention.
(1) The extracellular ferment liquor prepared in example 4 is concentrated for 0.5h under a 0.05MPa vacuum condition to obtain a concentrate, in which the content of ergothioneine is 0.2mg/mL.
(2) The following raw materials are weighed respectively: 200mL concentrated solution prepared in step (1), 100mL concentrated apple juice (with 65% content of soluble solids), 3g xanthan gum (stabilizer), 80g sucrose, 1g citric acid, 3g essence; and water is added to 1,000mL.
(3) The xanthan gum and sucrose are mixed homogeneously, and then the mixture is dissolved in a small amount of water; then the mixture is milled in a colloid mill to homogeneous state; next, the concentrate prepared in step (1), concentrated apple juice, citric acid and purified water are added in proportions specified in the formulation, the mixture is mixed homogeneously, and then the essence is added; next, the mixture is further milled in a colloid mill and homogenized in a homogenizer.
(4) The material obtained in step (3) is sterilized instantaneously at a high temperature of 130°C for 10s.
(5) The material treated in step (4) is packed in a sterile state.

### Example 11

This example is provided to describe the method for preparing a preparation (a functional beverage) in the present invention.
(1) The extracellular ferment liquor prepared in example 7 is sterilized for 20min at 121°C, and then is concentrated for 1h under a 0.1MPa vacuum condition to obtain a concentrate, in which the content of ergothioneine is 0.3mg/mL.
(2) The following raw materials are weighed respectively: 250mL concentrated solution prepared in step (1), 50g soy isolate protein (with 90wt% content of protein), 5g xanthan gum (stabilizer), 2g phospholipid, 20g sucrose, 0.25g aspartame, 1g essence; and water is added to 1,000mL.
(3) The xanthan gum, phospholipid, soy isolate protein and sucrose are mixed homogeneously, and then the mixture is dissolved in a small amount of water; then the mixture is milled in a colloid mill to homogeneous state; next, the concentrate, aspartame and purified water are added in proportions specified in the formulation, the mixture is mixed homogeneously, and then the essence is added; next, the mixture is further milled in a colloid mill and homogenized in a homogenizer.
(4) The material obtained in step (4) is sterilized instantaneously at a high temperature of 135°C for 6s.
(5) The material treated in step (5) is packed in a sterile state.

It can be seen from above examples: the method provided in the present invention doesn't require leaching and employs a simple process, and it makes the most of the ergothioneine in the extracellular ferment liquor.

While some preferred embodiments of the present invention are described above, the present invention is not limited to the details in those embodiments. Those skilled in the art can make modifications and variations to the technical scheme of the present invention, without departing from the spirit of the present invention. However, all these modifications and variations shall be deemed as falling into the scope of protection of the present invention.

In addition, it should be noted that the specific technical features described in above embodiments can be combined in any appropriate form, provided that there is no conflict. To avoid unnecessary repetition, the possible combinations are not described specifically in the present invention.

Moreover, different embodiments of the present invention can be combined freely as required, as long as the combinations don't deviate from the ideal and spirit of the present invention. However, such combinations shall also be deemed as falling into the scope disclosed in the present invention.

## Claims

1. A preparation containing ergothioneine, wherein the ergothioneine is provided by an extracellular ferment liquor that is obtained by removing the mycelia from ferment liquor produced through liquid fermentation of a mushroom, and/or a concentrate of the extracellular ferment liquor, the preparation comprises food, cosmetics and animal feed.

2. A preparation according to claim 1, wherein the mushroom is *Pleurotus ostreatus.*

3. A preparation according to claim 1 or 2, wherein the liquid culture medium used for the liquid fermentation contains 10-95g/L glycerol, 10-80g/L casein peptone, 1-6g/L KH₂PO₄, 0.2-5g/L MgSO₄, 3-45mmol/L methionine and 2-45mmol/L cysteine.

4. A preparation according to claim 3, wherein the liquid culture medium used for the liquid fermentation contains 65-95g/L glycerol, 40-80g/L casein peptone, 2-4g/L KH₂PO₄, 0.5-2g/L MgSO₄, 9-35mmol/L methionine and 5-20mmol/L cysteine.

5. A preparation according to claim 3 or 4, wherein the liquid culture medium used for the liquid fermentation further contains 0.5-50g/L tween.

6. A preparation according to any of claims 1-5, wherein the condition of liquid fermentation includes: fermentation temperature: 19-31°C, fermentation time: 7-15 days, and saturation of dissolved oxygen: 10-50%.

7. A preparation according to claim 6, wherein the condition of liquid fermentation includes: fermentation temperature: 23-28°C, fermentation time: 10-13 days, and saturation of dissolved oxygen: 15-40%.

8. A preparation according to any of claims 1-7, wherein the content of ergothioneine is 0.0005-50wt%, preferably 0.001-20wt%, based on the total weight of the preparation.

9. A method for preparing the preparation according to any of claims 1-8, comprising liquid fermentation of the mushroom, removing the mycelia from the obtained ferment liquor, and selectively concentrating the extracellular ferment liquor obtained after removing the mycelia.

10. A method according to claim 9, wherein the method for liquid fermentation of the mushroom comprises the following steps:
(a) inoculating *Pleurotus ostreatus* into a seed culture medium, and culturing for 3-6 days at 23-28°C to prepare a seed liquor, wherein the seed culture medium contains 25-40g/L maize meal, 15-35g/L bean cake powder, 30-80U/L alpha-amylase, 2-4.5g/L potassium dihydrogen phosphate, and 0.2-3g/L magnesium sulfate; and
(b) inoculating the seed liquor in an inoculum size of 4-20vol% into a liquid culture medium, and culturing for 7-15 days at 19-31°C, and controlling the saturation of dissolved oxygen at 10-50% during the culturing process, to obtain a ferment liquor that contains *Pleurotus ostreatus* mycelia, wherein the liquid culture medium contains 65-95g/L glycerol, 40-80g/L casein peptone, 2-4g/L potassium dihydrogen phosphate, 0.5-2g/L magnesium sulfate, 9-35mmol/L methionine and 5-20mmol/L cysteine.

11. A method according to claim 10, wherein the liquid culture medium further contains 0.5-50g/L tween.

12. Use of an extracellular ferment liquor obtained through liquid fermentation of a mushroom and/or a concentrate of the extracellular ferment liquor in preparing a preparation containing ergothioneine, wherein the preparation comprises food, cosmetics and animal feed.

13. Use according to claim 12, wherein the mushroom is *Pleurotus ostreatus.*
